Europäisches Patentamt

European Patent Office

Office européen des brevets

(11) Publication number: **0 100 373**
**A1**

(12) # EUROPEAN PATENT APPLICATION

(21) Application number: **82107061.2**

(22) Date of filing: **04.08.82**

(51) Int. Cl.³: **C 07 C 89/02**

(43) Date of publication of application:
**15.02.84 Bulletin 84/7**

(84) Designated Contracting States:
**BE DE FR GB IT NL SE**

(71) Applicant: **UNION CARBIDE CORPORATION**
**Old Ridgebury Road**
**Danbury Connecticut 06817(US)**

(72) Inventor: **Ahmed, Moinuddin**
**908 Mound Avenue**
**South Charleston West Virginia(US)**

(72) Inventor: **Nelson, James Russel**
**1002 Dell Way**
**South Charleston West Virginia(US)**

(72) Inventor: **Gibson, Charles Arnold**
**2910 Macon Street**
**South Charleston West Virginia(US)**

(74) Representative **Schmied-Kowarzik, Volker, Dr. et al,**
**Patentanwälte Dr. V. Schmied-Kowarzik Dipl -Ing. G**
**Dannenberg Dr. P. Weinhold Dr. D. Gudel Dipl.-Ing. S.**
**Schubert Dr. P. Barz Siegfriedstrasse 8**
**D-8000 München 40(DE)**

(54) Improved process for preparing alkanolamines.

(57) A process is provided for preparing alkanolamines
having a high yield of monoalkanolamine which comprises
reacting an alkylene oxide having from two to four carbon
atoms with ammonia in a molar ratio of ammonia to alkylene
oxide within the range from about 15:1 to about 50:1 at
temperatures at which the reaction proceeds and at press-
ures high enough to maintain the reaction mixture in a single
supercritical fluid phase.

EP 0 100 373 A1

COMPLETE DOCUMENT

## BACKGROUND OF THE INVENTION

This invention relates to a process for the preparation of alkanolamines and, more particularly, to a process for preparing alkanolamines with high yields of monoalkanolamine that may be run continuously by the reaction of alkylene oxides with a large excess of ammonia wherein the reaction mixture is maintained in a single phase as a supercritical fluid.

It is known that alkanolamines which are used in a variety of commercial applications such as emulsification agents for soaps and cosmetics and as a starting material for the production of raw materials for detergents, wetting agents, emulsifiers, textile auxiliaries and the like can be obtained by the reaction of alkylene oxides with ammonia or amines, the yield of alkanolamines being a mixture of mono; di-; and trialkanolamaines with, generally equal relative proportions of the three alkanolamines being frequently obtained. The relative proportions of these three alkanolamines in the product mixture, however, are known to depend on the relative quantities of alkylene oxide and ammonia that are reacted and methods have been used or suggested for achieving higher yields of one or more of the alkanolamines in the mixture by varying the proportion of reactants, such as by increasing the amount of ammonia relative to the alkylene oxide to obtain increased yields of monoalkanolamine, as well as by other process changes.

There is disclosed, for example, in U.S. Patent No. 2,196,554 to H. M. Guinot a process for preparing mono-hydroxylalkylamines with yields of 90%-95% by reacting at least 30 parts by weight of ammonia with one part of alkylene oxide. Relatively dilute aqueous ammonia solutions are employed and the patent discloses that steam generated during concentration of the reaction mixture is used for heating subsequent reaction mixtures of aqueous ammonia and alkylene oxide to reduce the heat energy requirements for the process. Another process for preparing alkanolamines with extremely high yields of monoalkanolamines and only small amounts of the di- and trialkanolamines by reacting alkylene oxide with large excess amounts of ammonia in a liquid phase reaction system is disclosed in U.S. Patent No. 3,697,598 to Weibull et al. The molar ratio of ammonia relative to alkylene oxide used in the process is within the range of 10:1 to 80:1 and the reaction is carried out in the presence of a cation exchange resin catalyst. The process of the patent is described as being a continuous process which is capable of being run isothermally or, preferably, adiabatically at temperatures in the range of from 20°C. to 250°C. when pressures are employed that are high enough to keep the reactants and reaction products in the liquid phase throughout the reaction. There is, however, no disclosure either in the description or in the examples of the patent which show that high yields of alkanolamines of any type are obtained when the process is carried out either adiabatically or isothermally without the use of cation exchange resin catalysts, and patentees state that without a cation exchange catalyst it is not possible to realize an adiabatic reaction because it is too slow. Further, in U.S. Patent No. 3,723,530 to Goetze et al., there is also disclosed a process for preparing a mixture of alkanolamines by the liquid phase reaction of ethylene oxide and a large excess of ammonia, that is mole ratios of ammonia to ethylene oxide of from 14 to 40 to one. The patent teaches that when the reaction is carried out in the presence of up to 1 mole of diethanolamine per mole of ethylene oxide, the product obtained will be a mixture of only monoethanolamine and triethanolamine with little or no diethanolamine being present. While the process of the invention is described as being capable of being run continuously either isothermally or adiabatically, the ammonia is usually employed in the form of an aqueous solution, the reaction is carried out in the liquid phase at temperatures in the range of from 60° to 150° and pressures of from 20 to 120 atmospheres, and the monoethanolamine content of the product mixture generally does not exceed 70 percent by weight.

While the processes heretofore disclosed suggest that they are suitable for use in preparing monoethanolamines in high yields by reacting alkylene oxides with excess amounts of ammonia, their usefulness in either batch or continuous operations depends on the presence of catalysts or supplemental process steps. It would be highly desirable, however, if a process was available which could be used to readily prepare monoalkanolamines at practical reaction rates that did not involve the potential additional problems associated

- 5 -

with catalysts or costs due to complicated or supplemental process steps.

## SUMMARY OF THE INVENTION

In accordance with the present invention, there is provided a process for preparing alkanolamines with high yields of monoalkanolamine which comprises reacting an alkylene oxide having from two to four carbon atoms with ammonia in a molar ratio of ammonia to alkylene oxide within the range from about 15:1 to about 50:1 at temperatures at which the reaction proceeds above about 100°C. and at pressures high enough to maintain the reaction mixture in a single supercritical fluid phase to form a product mixture containing predominantly monoalkanolamine. Unreacted ammonia, which is separated from the reaction mixture, may be recycled if desired.

The temperatures employed for carrying out the reaction are preferably as high as possible so that the reaction will proceed at a suitable rate, and temperatures above the critical temperature of the reaction mixture may be advantageously used. The pressure should be high enough to maintain the reaction mixture in a single nomogenous supercritical fluid phase at any point during the process. The density of the reaction mixture, which is dependent upon the pressure employed at the reaction temperature and is an important consideration as to the rate at wnich the reaction proceeds, should be maintained as high as possible and generally should be at least about 15 lbs./cu. ft. (240 kg/cu.m.). The reaction can be carried out batchwise or

- 6 -

continuously under isothermal or adiabatic conditions and, while no catalyst is required, the presence of a small amount of water in the reaction mixture has an advantageous catalytic effect. The term "supercritical fluid" as used herein is defined as the physical state of the reaction mixture wherein either the pressure or both the temperature and pressure conditions are above the critical values therefor.

## DESCRIPTION OF THE INVENTION

The process of the invention comprises reacting an alkylene oxide having from two to four carbon atoms with ammonia in a molar ratio of ammonia to alkylene oxide within the range from about 15:1 to about 50:1 at a temperature at which the reaction proceeds above about 100°C. and at pressures high enough to maintain the reaction mixture in a single supercritical fluid phase for the time necessary to form a product mixture composed predominately of monoalkanolamine (generally about 75%) and small amounts of di-and trialkanolamine and separating unreacted ammonia therefrom. The mono- di-, and trialkanolamines can also be separated if desired.

The alkylene oxides to which the process of the present invention is applicable is any 1,2-alkylene oxide having from two to four carbon atoms, including ethylene oxide, propylene oxide, 1,2-butylene oxide, 2,3-butylene oxide, and isobutylene oxide.

In accordance with the present invention, it is essential that a large excess of ammonia is used in the reaction to obtain yields of monoalkanolamines of at least 65 weight percent. It is advantageous to use about 15 to about 50 moles, and preferably from about 20 to about 35 moles, of ammonia for each mole of alkylene oxide to obtain yields in many cases of from about 70 to 80 weight percent. The ammonia should be added to the reaction mixture in a liquid state, generally in a substantially anhydrous condition. The liquid ammonia and alkylene oxide may be premixed just prior to feeding into the reaction vessel or each may be added separately to the reactor.

In accordance with the practice of the invention, it is important that the reaction of alkylene oxide and ammonia be carried out with the reaction mixture in a homogenous, single supercritical fluid phase so that the reaction will proceed at a suitable rate. The reaction can be carried out under isothermal or adiabatic conditions. The temperature at which the reaction should be carried out is within the range from about 100°C. to about 200°C., though the upper limit of the temperature is not critical. Preferably, the reaction temperature is within the range from about the critical temperature of the reaction mixture (generally from about 130°C.) to about 180°C. Under isothermal conditions, since the reaction is strongly exothermic, it is necessary to withdraw heat from the reaction mixture to keep the temperature approximately constant.

In the case when the reaction is to be carried out under adiabatic or nearly adiabatic conditions, the reactants are preheated to from about 100°C. to 130°C.

before they are introduced into the reactor. Because of the reaction heat involved, any selected initial reaction temperature is rapidly increased and the initial reaction temperatures should be chosen so that the maximum desired temperature will be obtained during the period of residence of the reaction mixture within the reactor. The preferred maximum temperature is between about 170°C. and 180°C. though the higher the reaction temperature, the higher the pressure that is necessary to maintain the density of the reaction mixture as high as possible.

At such reaction temperatures, it is essential that the pressures imposed on the system are high enough to maintain the reaction mixture in a single supercritical fluid phase. In any case, the reaction pressure should be at least as high as the critical pressure of the reaction mixture at any point encountered during the process. Preferably, the pressures imposed on the system are within the range from about 170 to about 240 atmospheres. The latter is a practical upper limit and is not critical.

As pointed out hereinabove, it is important that the reaction mixture is maintained in a single phase as a supercritical fluid and that the density thereof is as high as possible so that the reaction will proceed at a suitable rate. The density of the reaction mixture should be above the critical density and, in general, at least 15 lbs/cu. ft. (240 kg/cu.m.). Preferably, the density of the reaction mixture should be maintained in the range of from about 21 to about 28 lbs/cu. ft. or even higher if practical. The mole ratio of ammonia and alkylene oxide reactants and the reaction temperature have a significant effect on the density of the reaction mixture. It is important, therefore, that the reaction pressures are maintained as high as is practical so that the reaction mixture is not only maintained in a single supercritical fluid phase but that the density of said mixture is as high as possible so that the reaction will proceed at a practical rate.

While it is not essential that the process of the invention be carried out in the presence of any catalyst, advantageous embodiments of the process of the invention may be carried out with a small amount of water incorporated in the reaction mixture along with the alkylene oxide and ammonia reactants. It has been found that the presence of small amounts of water in the reaction mixture has an advantageous catalytic effect on the reaction rate for forming alkanolamines though it does not appear to affect the yield of monoalkanolamine in the product mixture. The amount of water that is present is not critical, and only small amounts of water may achieve the catalytic affect that is desired. In general, from about 0.5 percent up to about 5 percent by weight of water based on the weight of the reaction mixture need be present. Amounts of water greatly i: excess of that which may be catalytically useful, however, should be avoided to limit the energy requirements needed to separate water from the product mixture.

After conclusion of the reaction, substantially

- 10 -

all of the alkylene oxide has been reacted and the unreacted ammonia may be separated from the product mixture by any means known in the art, such as by reducing the pressure to below that at which the ammonia is in a gaseous phase, so that the ammonia can be separated as a gas. The ammonia can then be recycled, if desired, by repressurizing to a liquid phase before mixing with alkylene oxide. The unreacted ammonia may also be separated from the product mixture by distilling under pressure. The alkanolamine analogues in the product mixture may also be separated by known distillation methods or the product mixture may be used as a starting material for the preparation of other organic amines.

As pointed out hereinabove, the process of the invention may be carried out batchwise or continuously, either under isothermal or adiabatic conditions. In an alternate embodiment of the process of the invention which is run continuously, the ammonia and alkylene oxide reactants in the molar ratios hereinabove described are continuously fed, either separately or, preferably, as a mixture, to a tubular reactor which is capable of operating as efficiently as possible as a plug-flow reactor having means for providing the pressures needed to maintain the reaction mixture in a single supercritical fluid phase. The reactor may be carried out isothermally in a tubular reactor having cooling means or, advantageously, under adiabatic conditions where the reactants are preheated to a temperature, for example, between about 100°C. to

- 11 -

130°C. Small amounts of water may also be added to the reaction mixture, if desired.

The residence time of the single phase supercritical fluid reaction mixture in said adiabatic reactor should be sufficiently high to permit the reaction to proceed to completion, generally in less than about 1/2 hour. At the completion of the reaction, that is generally when all the alkylene oxide nas been reacted, the unreacted ammonia is separated from the product mixture as hereinabove described and recycled to the reactor. The recycled ammonia is pressurized to a liquid state prior to mixing within tne alkylene oxide and fresh make-up ammonia. The product mixture which is obtained can be separated into alkanolamine components by distillation methods known in the art or can be used as a starting material for the production of materials such a organic polyamines.

This invention will become more clear when considered together with the following examples which are set forth as being merely illustrative of the invention and which are not intended in any manner, to be limitative thereof. Unless otherwise indicated, all parts and percentages are by weight.

### Example 1

A 2 liter (1984 ml.) stainless steel autoclave having a high speed agitator and equipped with charging, sampling, and temperature control means was used in carrying out the reaction runs of this example. A series of reactions were run using liquid anhydrous ammonia, water, and ethylene oxide in the proportions reported in Table I. The ammonia and water were charged to the autoclave which was evacuated to a pressure of about 1 mm Hg absolute and then witn vigorous agitation were heated to 170°C. The ethylene oxide was then charged to the autoclave and, with vigorous agitation, the reaction temperature was maintained at 170°C. for 30 minutes. A sample of tne reaction mixture was taken after the time indicated in Table I during each of the reaction runs of tnis example. After 30 minutes, the reaction mixture was cooled to below 50°C. and unreacted ammonia was vented from the autoclave until tne pressure in the autoclave indicated essentially complete removal of gas. The liquid product mixture was then drained from the autoclave and the composition thereof was determined by gas chromatographic analysis.

The amount of ammonia and ethylene oxide reactants used were intended to obtain an average ammonia to ethylene oxide mole ratio of 25:1 for each of the reaction runs of tnis example. The average density of the reaction mixture during each of the reaction runs of this example was 24 lbs./cu. ft.

A summary of the proportion of ingredients, reaction conditions and composition of tne product mixtures for each of the reaction runs of this example are reported in Table I.

It is apparent from results shown in Table I that an alkanolamine mixture was prepared with a high yield of monoalkanolamine during each of the runs of this example.

TABLE I

| Run | Ammonia Moles | Grams | Ethylene Oxide Moles | Grams | Water Moles | Grams | Sample Time (Min) | Press. PSIG | Ethylene Oxide Residual Mol % | Product Distribution Wt %(a) MEA | DEA | TEA |
|---|---|---|---|---|---|---|---|---|---|---|---|---|
| 1 | 39.81 | 678 | 1.57 | 69.1 | 0.754 | 13.57 | 0 | 3250 | - | 73.21 | 22.04 | 4.74 |
|   |   |   |   |   |   |   | 15 | 2850 | 0.012 |   |   |   |
| 2 | 39.81 | 678 | 1.57 | 69.1 | 0.754 | 13.57 | 0 | 3310 | - | 75.53 | 20.20 | 4.27 |
|   |   |   |   |   |   |   | 15 | 2800 | 0.012 |   |   |   |
| 3 | 39.81 | 678 | 1.62 | 71.4 | 0.754 | 13.57 | 0 | 3300 | - | 75.24 | 20.34 | 4.42 |
|   |   |   |   |   |   |   | 12 | 2800 | 0.041 |   |   |   |
| 4 | 39.81 | 678 | 1.62 | 71.3 | 0.754 | 13.57 | 0 | 3210 | - | 74.54 | 20.90 | 4.55 |
|   |   |   |   |   |   |   | 12 | 2800 | 0.025 |   |   |   |
| 5 | 39.81 | 678 | 1.59 | 70.0 | 0.754 | 13.57 | 0 | 3500 | - | 75.36 | 20.17 | 4.47 |
|   |   |   |   |   |   |   | 9 | 2820 | 0.096 |   |   |   |
| 6 | 39.81 | 678 | 1.57 | 69.1 | 0.754 | 13.57 | 0 | 3500 | - | 76.12 | 19.96 | 3.89 |
|   |   |   |   |   |   |   | 9 | 2950 | 0.14 |   |   |   |
| 7 | 39.81 | 678 | 1.58 | 69.5 | 0.754 | 13.57 | 0 | 3410 | - | 74.68 | 21.04 | 4.28 |
|   |   |   |   |   |   |   | 6 | 2800 | 0.55 |   |   |   |
| 8 | 39.81 | 678 | 1.62 | 71.3 | 0.754 | 13.57 | 0 | 3450 | - | 76.03 | 19.96 | 3.99 |
|   |   |   |   |   |   |   | 3 | 2900 | 1.74 |   |   |   |
| 9 | 39.81 | 678 | 1.62 | 71.3 | 0.754 | 13.67 | 0 | 3250 | - | 75.08 | 20.44 | 4.45 |
|   |   |   |   |   |   |   | 3 | 3050 | 1.96 |   |   |   |
| 10 | 39.81 | 678 | 1.59 | 70.1 | 0.754 | 13.57 | 0 | 3300 | - | 73.21 | 22.04 | 4.74 |
|   |   |   |   |   |   |   | 25 | 2700 | - |   |   |   |

(a)After a total reaction time of 30 minutes.

- 13 -

## Example 2

Using the apparatus and procedure of Example 1, a series of reaction runs were carried out to demonstrate tne effect of reaction product density and ammonia to ethylene oxide mole ratio on the reaction rate and distribution of alkanolamines in the product mixture. Runs 1 to 8 were carried out with an average ammonia to etnylene oxide mole ratio of 30 to 1 ana runs 9 to 12 use a mole ratio of 25 to 1. Runs 1 to 4 and 9 to 12 were carried out at an average density of 22 lbs/cu. ft and Runs 5 to 8 were carried out at an average density of 24 lbs/cu. ft. A sample of the reaction mixture was taken from the reactor during each reaction run.

The proportion of ingredients, temperature and pressure condition's and analysis results (used analytical procedures described in example 1) for eacn of the reaction runs of this Example are summarized in Table II.

It is apparent from the results snown in Table II that an alliauolamine product mixture containing high yields of morroethanolamine was prepared during eacn reaction run of this example. The reaction rate for each of the runs would be suitaole but the results snow that the reaction rate for runs 1 to 4 which were maintained at an average density of 22 lbs/cu. ft. was somewhat slower than the reaction rate for runs 5 to 8 which were maintained at an average density of 24 lbs/cu.ft.

TABLE II

| Run | Ammonia Moles | Grams | Ethylene Oxide Moles | Grams | Water Moles | Grams | Density lbs/ft3 | Time (Min) | Temp. °C | Press. PSIG | E.O.R. Mol-%* | Product Distribution Wt% [a] MEA | DEA | TEA |
|---|---|---|---|---|---|---|---|---|---|---|---|---|---|---|
| 1 | 37.05 | 631 | 1.20 | 52.8 | 0.70 | 12.6 | 21.89 | 0 | 169 | 2900 | — | | | |
|   |       |     |      |      |      |      |       | 4 | 170 | 2800 | 1.59 | 75.97 | 19.37 | 4.66 |
| 2 | 37.05 | 631 | 1.23 | 54.4 | 0.70 | 12.6 | 21.94 | 0 | 170 | 3020 | | | | |
|   |       |     |      |      |      |      |       | 8 | 173 | 2860 | 0.53 | 76.01 | 19.30 | 4.69 |
| 3 | 37.05 | 631 | 1.26 | 55.44 | 0.70 | 12.6 | 21.98 | 0 | 171 | 3050 | — | | | |
|   |       |     |      |       |      |      |       | 14 | 170 | 2600 | 0.11 | — | — | — |
| 4 | 37.05 | 631 | 1.23 | 54.4 | 0.70 | 12.6 | 21.94 | 0 | 170 | 2800 | — | | | |
|   |       |     |      |      |      |      |       | 18 | 170 | 2560 | 0.07 | 81.02 | 15.57 | 3.4 |
| 5 | 40.42 | 688 | 1.36 | 59.8 | 0.74 | 13.7 | 23.95 | 0 | 170 | 3100 | — | | | |
|   |       |     |      |      |      |      |       | 4 | 172 | 3100 | 1.14 | 76.12 | 19.24 | 4.64 |
| 6 | 40.16 | 684 | 1.40 | 61.6 | 0.74 | 13.7 | 23.87 | 0 | 170 | 3300 | — | | | |
|   |       |     |      |      |      |      |       | 8 | 172.5 | 2940 | 0.16 | — | — | — |
| 7 | 40.98 | 698 | 1.32 | 58.1 | 0.74 | 13.7 | 24.2 | 0 | 170 | 3450 | — | | | |
|   |       |     |      |      |      |      |      | 12 | 171 | 3240 | 0.03 | 82.67 | 14.56 | 2.76 |
| 8 | 40.42 | 688.4 | 1.35 | 59.4 | 0.74 | 13.7 | 23.94 | 0 | 170 | 3350 | — | | | |
|   |       |       |      |      |      |      |       | 14 | 171 | 3000 | 0.015 | 76.97 | 18.94 | 4.09 |
| 9 | 36.41 | 620 | 1.45 | 63.8 | 0.69 | 12.4 | 21.89 | 0 | 181 | 3700 | — | | | |
|   |       |     |      |      |      |      |       | 4 | 180 | 3000 | 0.61 | 73.32 | 21.24 | 5.44 |
| 10 | 36.41 | 620 | 1.45 | 63.7 | 0.69 | 12.4 | 21.88 | 0 | 179 | 3400 | — | | | |
|    |       |     |      |      |      |      |       | 8 | 179 | 2950 | 0.13 | — | — | — |
| 11 | 36.76 | 626 | 1.46 | 64.2 | 0.69 | 12.4 | 22.09 | 0 | 184 | 3700 | — | | | |
|    |       |     |      |      |      |      |       | 12 | 178 | 2980 | 0.015 | 74.91 | 20.2 | 4.89 |
| 12 | 36.41 | 620 | 1.45 | 64.0 | 0.69 | 12.4 | 21.89 | 0 | 180 | 3625 | — | | | |
|    |       |     |      |      |      |      |       | 16 | 179.5 | 3010 | 0.01 | 74.07 | 20.78 | 5.15 |

[a] After a total reaction time of 30 minutes    *) Ethylene Oxide Residual

- 16 -
Example 3

A mixture of 786.4 grams (46.2 moles) of ammonia and 15.7 grams of water was charged to tne stirred autoclave described in Example 1 which had previously been vacuated to about one millimeter of Hg vacuum. The mixture was heated to 170°C. and 3900 psig with stirring and 54.1 grams (1.23 moles) of ethylene oxide were injected into the stirred mixture. The density of the reaction mixture was maintained at an average of 27 lbs./cu. ft.

Small samples of the reaction mixture were periodically removed from the autoclave and analyzed by mass spectrometry for residual ethylene oxide and tne sample times and conversion of ethylene oxide are shown below in Table III.

TABLE III

| Sample Number | Time, Minutes from EO Injection | % Conversion Based on Residual EO |
|---|---|---|
| 1 | 3 | 74.9 |
| 2 | 6 | 77.2 |
| 3 | 9 | 92.3 |
| 4 | 24 | 100.0 |

After 30 minutes from the time ethylene oxide was added, the mixture in the autoclave was cooled to below 50°C. and the unreacted ammonia was separated from the product mixture. The liquid product mixture was discharged from the autoclave and analyzed for alkanolamine composition by gas chromatography. The product mixture was determined to contain 84.03 percent monoethanolamine, 14.36 percent diethanolamine, and 1.60 percent triethanolamine.

For comparison purposes, tne following reaction run was carried out in the autoclave reactor described in Example 1.

In this control, a mixture of 678 grams (39.81 moles) of ammonia and 13.57 grams of water was charged to the evacuated, stirred autoclave of Example 1 and heated to 120°C. Nitrogen gas was added to the autoclave until a pressure of 2000 psig was obtained. Ethylene oxide (72.98 grams, 1.659 moles) was injected into the stirred mixture in the autoclave and the temperature was maintained at 120°C. during the entire run. The reaction mixture in the autoclave was a single phase liquid.

Small samples were periodically removed from the autoclave reactor and analyzed by mass spectrometry for residual ethylene oxide and the results are summarized in Table V, below.

### TABLE V

| Sample Number | Time, Minutes from EO Injection | % Conversion Based on Residual EO |
|---------------|--------------------------------|-----------------------------------|
| 1 | 5 | 16.25 |
| 2 | 10 | 33.00 |
| 3 | 15 | 35.25 |
| 4 | 20 | 56.00 |
| 5 | 25 | 73.00 |
| 6 | 30 | 82.75 |
| 7 | 40 | 92.50 |

The reaction was continued at 120°C. for an additional 138 minutes after which the mixture was cooled, unreacted ammonia was separated therefrom, and the liquid product mixture was recovered and analyzed by gas chromatography.

The product mixture was determined to contain 74.44 percent of monoethanolamine, 21.17 percent diethanolamine, and 4.38 percent triethanolamine. While the liquid phase reaction resulted in high yields of monoethanolamine, the reaction rate was determined slow to be suitable.

## WHAT IS CLAIMED IS:

1. A process for preparing alkanolamines having a high yield of monoalkanolamine which comprises reacting an alkylene oxide having from two to four carbon atoms with ammonia in a molar ratio of ammonia to alkylene oxide within the range from about 15:1 to about 50:1 at temperatures at which the reaction proceeds above about 100°C., and at pressures high enough to maintain the reaction mixture in a single supercritical fluid phase to form a product mixture containing predominantly monoalkanolamine.

2. The process of claim 1 wherein the density of the reaction mixture is above 15 lbs./cu. ft., preferably in the range from about 21 to about 28 lbs./cu. ft.

3. A process according to any one of the preceding claims wherein the reaction is carried out in the presence of a small, catalytically effective amount of water, preferably in the presence of from about 0.5 to 5 percent by weight of water based on the weight of the reaction mixture.

4. A process according to any one of the preceding claims wherein the reaction temperature is up to about 200°C, preferably in the range from about the critical temperature of the reaction mixture to about 180°C.

- 2 -

5. A process according to any one of the preceding claims wherein the reaction is carried out at pressures in the range from about 170 to 240 atmospheres.

6. A process according to any one of the preceding claims wherein the process is carried out adiabatically.

7. A process according to any one of the preceding claims wherein unreacted ammonia is separated from the product mixture.

8. The process of claim 7 wherein a product mixture containing predominately monoalkanolamine is recovered.

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | US-A-3 697 598 (WEIBULL et al.) * Table 2, comparative example : claims * & DE-A-1 941 859 | 1-8 | C 07 C 89/02 |
| | --- | | |
| A | Soviet Inventions Illustrated Week C24, 23 July 1980, section E16 & SU-A-682 508 | | |
| | ----- | | |

TECHNICAL FIELDS
SEARCHED (Int. Cl. 3)

C 07 C 89/00

The present search report has been drawn up for all claims

| Place of search | Date of completion of the search | Examiner |
|---|---|---|
| BERLIN | 21-03-1983 | BREW C.H. |